# EUROPEAN PATENT SPECIFICATION

(11) **EP 3 178 454 B1**
(45) Date of publication and mention of the grant of the patent: **31.10.2018**
(21) Application number: 16202639.7
(22) Date of filing: 07.12.2016
(51) Int. Cl.: A61F 9/06, B23K 9/32

(54) **METHOD AND SYSTEM FOR ACCELERATED REACTION OF THE DARKENING OF THE OPTICAL ELEMENT IN A WELDING PROTECTION DEVICE**
VERFAHREN UND SYSTEM FÜR DIE BESCHLEUNIGTE VERDUNKELUNG EINES OPTISCHEN ELEMENTS IN EINER SCHWEISSSCHUTZVORRICHTUNG
MÉTHODE ET SYSTÈME POUR L'OBSCURCISSEMENT ACCÉLÉRÉ D'UN ÉLÉMENT OPTIQUE D'UN MASQUE DE PROTECTION DE SOUDAGE

(30) Priority: 07.12.2015 SK 501162015 U; 30.05.2016 SK 500312016; 30.05.2016 SK 500712016 U
(43) Date of publication of application: 14.06.2017
(73) Proprietor: Trafimet Group S.p.A., 36020 Castegnero (VI) (IT)
(72) Inventor: Papp, Róbert, 927 05 Dlhá nad Váhom (SK); Lipták, Kristián, 925 22 Velke Úlany (SK); Lauko, Robert, 811 01 Bratislava - Staré Mesto (SK)
(74) Representative: Ziliotto, Tiziano

(56) References cited:
- US-A- 4 638 146
- US-A1- 2013 128 135
- US-B1- 6 734 393

## Description

### Field of technology

The invention concerns the shortening of the reaction time of the self-darkenable optical element in the personal protection device, for example welding helmet, where the optical element darkens in reaction to the intensive luminous manifestation of the running technological process, for example in reaction to the lit electric art during welding, cutting and so on.

### Prior state of the art

Personal protective devices such as self-darkenable goggles, shields, helmets, etc. are used in order to protect the sight during welding, cutting and similar processes which are accompanied with the intensive light. Essential part of such protective elements is an optical element capable of changing the permeability of light on the basis of the instruction from the controlling circuit (automatic darkening filters ADF). During electric arc there is an intensive ultraviolet, infrared and visible light radiation. The sensor sensible to the mentioned luminous manifestation issues an instruction to dim or darken the optical element and the darkened optical element prevents the transition of the harmful elements of the radiation to the sight.

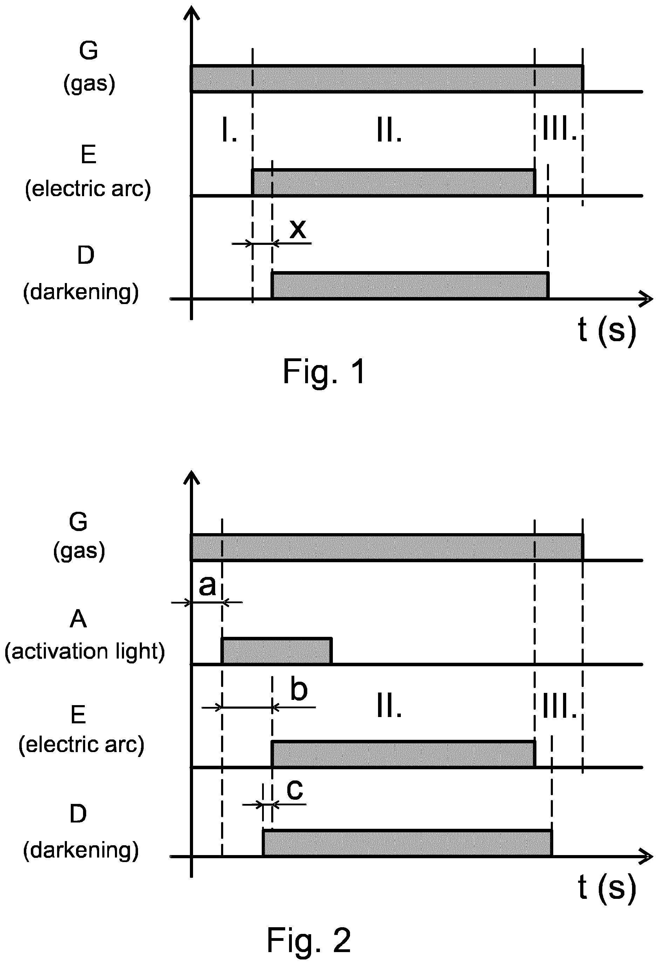

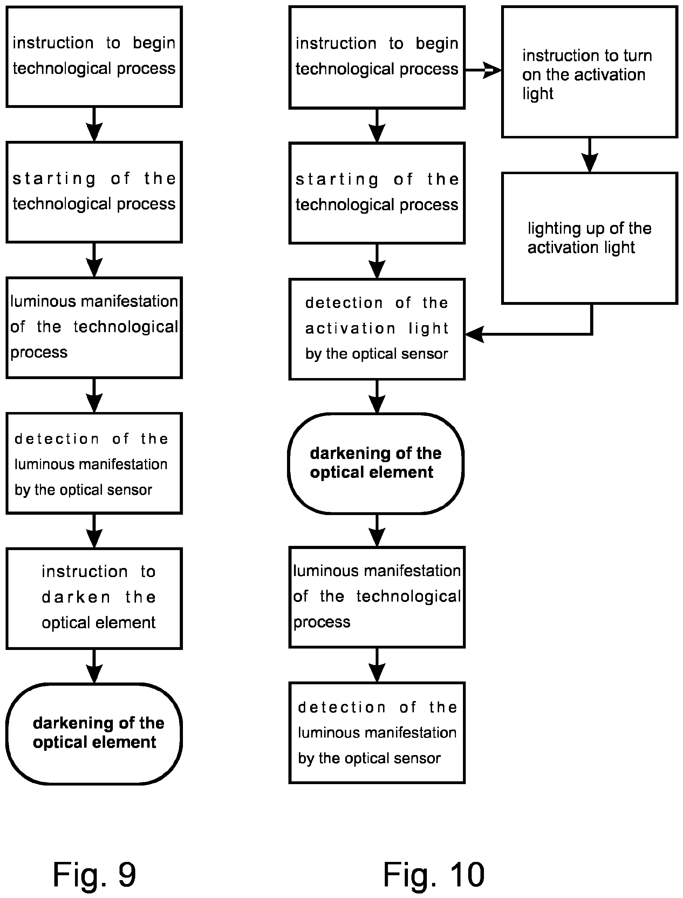

In order to achieve reliable protection of the sight it is necessary that the darkening of the optical element takes place as soon after the detection of the dangerous luminous phenomenon as possible, for example after the ignition of the electric arc during welding. In order to fulfill this technical task there are multiple solutions, for example publications WO2005009309A1, WO2011097841A1, US8264265B2, US2013128135A1 which shorten the time of the detection of the dangerous luminous manifestation and shorten the reaction time of the optical element itself; that is, they shorten the time needed by the optical element to achieve the optical change. These two stages of detection and reaction last less than 0,3 ms; some quality welding helmets are able of darkening in 0,05 ms (1/20 000 s) after the ignition of the electric arc (figures 1 and 9). In absolute numbers these times are short and it is problematic to shorten them more, but in order to improve the protection of the sight it is preferable to achieve zero reaction time.

At the beginnings of the development of the personal protective devices with the automatic darkening element 30 years ago there have been efforts to quicken the reaction. Patent US 4,638,146 tackled slow, unreliable detection of the luminous manifestation by using a magnetic field detector to detect the beginning of the welding, whereby the signal to darken the optical element is transmitted optically by means of an infrared transmisison channel. The protective device on its own, however, as an independent element, is not able to detect the luminous manifestation of the welding in this solution at all, and individual stages of the detection and transmission in the chain of the instructions arranged one after another; that is why such method did not become widespread. It has been surpassed when the optical sensors were sped up for the first time.

Publications US2003206491A1, WO2008082751A1 disclose the direction of the welding helmet by the voice; welder can darken the optical element in advance by voice command. Other solutions work on similar principle, where there is a control button or similar element which can darken even before the electric arc is ignited. Such solutions are however uncomfortable and they require continuous issuing of commands which runs contrary to the demand for the automatized operation.

Patent US 7,812,279 B2 offers improvement, where the welding helmet is controlled by the command from the welding apparatus during welding with the protective atmosphere. Electric arc is in case of such sort of welding ignited with a delayed activation of the trigger of the welding gun. This delay is in fractions of second and it is intentional, so that the environment of the weld is supplied by the protective gas from the pressure vessel. This allows to issue a command to darken the welding helmet before the ignition of the electric arc. In case of the invention according to publication US 7,812,279 B2 the command to darken is issued to the helmet by the radio transmission. This requires that the helmet has a receiving element for the communication with the welding apparatus. Such solution therefore cannot be used with the existing self-darkening helmets (as in US 6,734,393). The use of the radio transmission between transmitter and receiver on the helmet is problematic; there is strong electromagnetic field during welding which can hamper the correct transmission. In the working places where multiple simultaneous weldings occur there can be complications with the interference of multiple radio frequency signals. The high energy demands are disadvantageous, too, as is the overall complexity of the receiving circuit which will require relatively powerful independent source of energy.

Such solution is desired and not known which will be simple, which will have low energy demands, which could be used for all sorts of protective devices, which would shorten the time between the creation of the luminous phenomenon and the darkening of the optical element and which will be comfortable for the personnel to wear and operate.

### Essence of the invention

The abovementioned deficiencies in the prior state of the art are remedied by the system according to claims 1 to 9 and method according to claims 10 to 16.

The abovementioned deficiencies are significantly remedied by a method according to claims 10 to 16 of an accelerated reaction of a darkening of the optical element in the personal protective device for the protection of the sight, where the optical sensor on the protective device detects the luminous manifestation of the technological process and on the basis of the signal from the optical sensor the darkening of the optical element is activated, which by its darkening limits the permeation of the undesired radiation from the luminous manifestation to the sight according to this invention, which essence lies in the fact that within the device realizing the technological process an instruction (command) to begin such technological process is detected, an activation light is subsequently turned on on the basis of such instruction which shines in the direction of the optical sensor in such a way that the activation of the optical sensor by means of the activation light takes places before the optical sensor detects the luminous manifestation of the technological process itself. Activation of the optical sensor means a recording of the light by the optical sensor, which results in the issuing of the instruction or command to darken the optical element.

The term "technological process" in this file denots mainly an electric arc welding, where an electric arc is produced during the transfer of the current between the electrode and the welded material, which includes an electric arc cutting as a specific case of adjustement of the parameters.

Hitherto known cycle of the automatic darkening of the optical element is following, if we simplify:
1. instruction to begin technological process
2. starting of the technological process
3. luminous manifestation of the technological process
4. detection of the luminous manifestation by the optical sensor
5. instruction to darken the optical element
6. darkening of the optical element.

We see that in the first three steps in the prior state of the art the protective device is only in the standby mode, it does not realize any activity. In the solution according to this invention the control of the protective device begins with the first step, which gives us crucial time needed for the darkening:
1. instruction to begin technological process + instruction to turn on the activation light
2. starting of the technological process + lighting up of the activation light
3. detection of the activation light by the optical sensor
4. luminous manifestation of the technological process + darkening of the optical element
5. detection of the luminous manifestation by the optical sensor; the optical element remains darkened.

It suffices if the activation light is on until the time when the optical element of the protective device detects the luminous manifestation from the technological process itself. The activation light will usually overlap for a short period with the detection of the luminous manifestation and then the activation light will be turned off in order to lower overall energy demands. After the detection of the luminous manifestation by the optical sensor on the personal protective device, the activation light can turn off; the instruction derived by the original control of the protective device is sufficient to darken the optical element. If the activation light will last for unnecessarily long, it could cause darkening of the optical element at time when the technological process itself - accompanied by the luminous manifestation - finishes. In any case the activation light should shine at least for the period which corresponds to the lead time, advance ignition of the activation light before the moment of the detection of the luminous manifestation of the technological process. The value of the temporal overlap of the shining of the activation light with the luminous manifestation can be adjusted by the control element, usually the total period of the shining of the activation light will not be longer than 2 to 5 s.

Compared to the prior state of the art according to US 4,638,146, the method according to this invention takes place in parallel with the commont, not adjusted protective device, which has its own detection of the luminous manifestation and this detection is still active and used in the process. As opposed to the prior state of the art, the activation light according to this invention thus does not have to shine continuously during the whole duration of the luminous manifestation. The difference also lies in the succession of the steps, where the instruction to light up the activation light takes place before the transmisison of the current between the electrode and the welded material. The transmission of the current without delay causes the luminous manifestation.

In principle, the method can be adjusted and changed in such a way that the activation light shines for a longer timae - even during the whole duration of the welding - which is mainly beneficial in the case of hidden welding, where a direct optical route between the place of welding and optical sensor of the protective device is lacking.

In order to improve the coordination of the period of shining of the activation light with the technological process running, an arrangement will be preferable where the control of the activation light is capable of recognizing the creation of the luminous manifestation - for example - directly by its own, second optical sensor or indirectly by the change in the electric current in the electric arc. Such recognition is not necessary for the activation light to function; activation light can be simply turned off after - for example - one second from the lighting up, but there are work operations (for example, short spot or line welds) where a long period of the activation light will, in fact, overlap with the ending of the given phase of the technological process. In such case the optical element would have been darkened despite the ending of the luminous manifestation. If the control of the activation light can recognize the beginning of the luminous manifestation, the activation light can be turned off instantly, because the darkening of the optical element will be secured on the basis of the sensing of the luminous manifestation.

Recognition of the luminous manifestation for the purposes of turning off of the activation light can be managed by the second optical sensor placed, for example, in the vicinity of the activation light by the body of the welding gun. The term "welding gun" denotes a welding torch which welds, that is, it mainly defines the place of welding. The optical sensor for the recognition of the luminous manifestation is in the description denoted as "second" only in order to distinguish it from the necessary optical sensor on the protective device itself. The instruction to turn off the activation light after the creation of the luminous manifestation can in another arrangement according to this invention arise from the current meter in the supply cables of the welding gun, or such instruction can be part of the control of the automatized welding workplace. The shortening of the period of shining of the activation light lowers energy demands and increases the longevity of the used battery or accumulator.

Time period between the instruction to begin the technological process and its luminous manifestation can be different based on the particular type of the technological process. In case of the welding with the non-melting electrode in the protective atmosphere of gases (TIG, WIG), or of the welding with the unwound electrode (MIG-MAG) in the protective atmosphere of CO₂, or CO₂ with argon (Metal Inert Gas, Metal Active Gas) the electric arc is intentionally ignited with the delay relative to the pressing of the trigger on the welding gun. This delay (pre-gas time) can be so long that the immediate lighting up of the activation light can cause too early darkening, which can be uncomfortable or confusing for the welder. The proposed solution can basically achieve negative reaction time of the darkening in response to the luminous manifestation. It will therefore be preferable if the command to initiate the activation light can be delayed after the command to initiate the technological process. Such delay will be adjustable in the preferable arrangement, usually up to 2 seconds. The method according to this invention is preferably used mainly during the welding with the protective atmosphere, where the welding process involves an intentional delay of the ignition of the electric arc, which is used to transfer a protective gas to the welding point. In principle, though, it is possible to use the method with other technological processes with th luminous manifestation, whereby the time delay between the instruction to begin the technological process and the passage of the current through the electrode is ensured.

In order to achieve balance between the reliable protection of the sight and the comfort of the welder it is preferable to achieve certain, very short negative reaction time between luminous manifestation and darkening of the optical element. It is technically problematic to stably achieve exactly zero reaction time and the setting with the positive reaction time does not use the potential of this invention adequately. In order to optimize the short negative reaction time an arrangement will be of use where the control of the activation light involves recognition of the luminous manifestation of the technological process. Such recognition can serve to turn off the activation light after the creation of the luminous manifestation as disclosed in this description. The control of the activation light can include the adaptation algorithm by which the control can learn to set the optimal value of the delay against the moment of the instruction to initiate the technological process. The adaptation can begin, for example, by setting the delay to zero at the first turning on of the activation light and then it can measure the time until the recording of the luminous manifestation. This time period, decreased by the small value of the lead time, will be used as a correction for the subsequent delay. The control of the activation light continually learns how long a time period - and with which statistical margin of error - lasts between the instruction to begin the technological process and its luminous manifestation. After certain period of inactivity which could signalize the transition to another workplace after the reset the adaptation mechanism starts anew. It is possible to use manual adjustment of the delay combined with the automatic adjustment based on the measurement of time.

If the system includes a second optical sensor, too, that is, a sensor of the luminous manifestation itself, a learning algorithm can be part of the method within a framework of which a time is analyzed between the pressing of the trigger of the technological process and a creation of the luminous manifestation. After measuring the time at the next pressing of the trigger it sends an emitting signal of the activation light just before the creation of the luminous manifestation, which saves the energy in the power supply of the activation light. The process of control of the activation light can be programmed as "multi-stroke" - for example two- or four-stroke - welding, where the welding machine reacts with ignition of the electric arc to one pressing of the trigger, whereby it keeps functioning until the second pressing of the trigger. The second pressing of the trigger causes switching off of the electric arc. It is possible to proceed in the point regime, where the welding machine initiates pulse welding with the discontinuous electric arc for the individual welding points. The second pressing of the trigger stops the point regime.

Some technological processes have work regimes which are initiated by pressing the trigger and then they continue until the next pressing of the trigger. The second pressing of the trigger is an instruction to end a given phase of the process. If the switch of the control of the activation light is connected with such a trigger - for example a trigger of the welding gun - the activation light at the end of the given phase will receive inappropriate signal to light up, which will not be dangerous but which will decrease the comfort of the welder. In case of regimes which begin with the first pressing of the trigger and continue until the second pressing of the trigger it is preferable to proceed in such a way that the activation light lightens up after each first pressing of the trigger and the control of the activation light will ignore each second pressing of the trigger. Such optional regime can be adjustable by the control of the activation light. Counting which pressing is first and which is second is usually adjustable.

It is possible to proceed with the same goal - that is, trying to leave out the activation light at the second pressing of the switch in the previously disclosed regime - in such a way that the control of the activation can recognize the ongoing technological process and then it can decide that pressing or holding of the switch during the ongoing phase of the technological phase does not mean an instruction to turn on the activation light. In order to recognize the ongoing technological process one can use second optical sensor connected with the control of the activation light.

There are technological processes - such as point welding - where after the pressing of the trigger, and thereby pressing of the switch, a phase of the process with the discontinuous presence of the luminous manifestation starts. In case the control of the activation light is connected with the control of the discontinuous process, the activation light will light up before each such independent creation of the luminous manifestation. In case the given device is wholly independent and disconnected from the control of the activation light, it will be preferable if the control of the activation light recognizes the repeating cycles of the luminous manifestation of the technological process. Such process can be part of the adaptation algorithm, where the control will turn on the activation light before the calculated creation of the luminous manifestation on the basis of the regularly repeating creation and extinction of the luminous manifestation (without pressing of the switch).

Aside from the entering of the instruction to darken before the creation of the luminous manifestation, the essential part of the proposed invention is the activation light, which the existing optical sensor of the protective device reacts to. Pursuant to the prior state of the art a cable transfer or radio-frequency channel can be used for the control of, for example, welding helmet - but this would require a new construction of helmet. Such helmet would be compatible only with the tools by the same producer and a solution would not be possible to use on the existing helmets. It is the creation of the activation light according to this description which simply solves all these problems. With help of the activation light we produce a one-direction communication channel with the optical sensor on the protective device, whereby no further modification or equipment of the protective device is needed. An existing optical sensor acts as a receiver in such one-direction communication channel. It suffices if the activation light is placed within the reach of the optical sensor of the protective device and this activation light will direct all sorts of the protective devices irrespective of their producers, since all such devices react to light.

Activation light does not have problems with interference in case of the placement in the proximity with other workplaces with the activation light. It holds that if the activation light permeates to the neighboring workplace, then the dangerous luminous manifestation of the technological process permeates it as well and that it is necessary to separate these workplaces better, otherwise the protective device would darken on the instructions from the neighboring workplace. It is therefore not necessary to code or modulate the signal from the activation light in any way.

The abovementioned deficiencies in the prior state of the art are significantly remedied by the system according to claims 1 to 9 for accelerated reaction of the darkening of the optical element in the personal protective device for the protection of the sight, which includes an optical sensor and an aperture with the optical element with the adjustable permeability for the purposes of limitation of the permeation of the radiation from the luminous manifestation to the sight, where the optical sensor for the detection of luminous manifestation is placed within the protective device and it is connected with the control of the optical element according to this invention, which essence lies in the fact that the system includes an activation light emitting a radiation which is detected by the optical sensor which within the personal protective device detects the luminous mnifestation of the electric arc welding. The activation light has a switch which is connected or designed to be connected with the trigger of the device for the production of the electric arc in such a way that the trigger is activated before the transfer of the current between the electrode and the welded material, whereby the activation light is placed within the reach of the optical sensor.

The activation light emits a radiation which falls within the sensitivity spectrum of the optical sensor in the protective device. Obviously, it should not be a dangerous ultraviolet radiation which a personnel should be protected from. Usually the activation light will be formed by at least one infrared LED diode. Optical sensors are sensitive to the infrared light and a low input power of the LED diode is already sufficient to activate the optical sensor. In order to achieve a desired effect a construction and power supply of the LED diode which is used in the remote controls suffices; power of the LED diode ranging from 0,1 to 2 W suffices. Such diodes are considered safe and harmless for the human health. Use of the activation light - for example in the form of the infrared LED diode - is, as if, emulation or deception of the detection of the luminous manifestation of the technological process. After the turning on of the activation light the optical sensor evaluates the situation as if the luminous manifestation already occurred and it issues an instruction to darken the optical element. In reality the luminous manifestation caused by the technological process - that is, a luminous manifestation with the dangerous elements of the radiation occurs after the lighting up of the activation light.

Abovementioned main features of the invention offer a person skilled in the art various options of realization with particular technical means. Activation light can be placed at various places on the workplace, whereby it should be within the reach of the optical sensor of the protective device. During the welding with the welding torch - so-called welding gun - which has a trigger, an arrangement proved preferable where the switch of the activation light is placed on the trigger of the welding gun or it is a part of this trigger and the activation light is placed directly on the gun, for example at the mouth of the gun. The welder holds the gun always in such a way that he or she sees it during the work, and the place of the luminous manifestation is located in the gun's proximity. It follows from this that the protective device - mainly a welding helmet - is with its optical sensor oriented in such a way that the sensor always has a welding gun in its field of view.

Even in case the workplace is automatized or robotized, the activation light can be placed on the welding torch, because there will be a luminous manifestation in its vicinity. An activation light can be placed in the fixed place on the production line, or multiple commonly controlled activation lights can be used. Thereby each worker who looks at a given workplace from the expected side has one of the activation lights in the field of view of the optical sensor of his or her protective device.

During the shining the activation light can be stably supplied by power or it can be frequentially supplied by the intermittent voltage. Some optical sensors have increased sensitivity to certain range of frequencies and because of this the system can include and adjusting (or adjustment) element for changing of the supply frequency of the activation light. In such case the activation light will not only have a particular wavelength, but also a particular emitting frequency. In case of frequentially intermittent power supply it is preferable if the frequency is within a range from 5 to 250 Hz. Power supply of the activation light, frequency generator, delay adjustment element and other electronic elements necessary for the control of the activation light can be in this description and in the claims together name as control electronic, or control unit.

The wavelength of the LED diode is basically stable; if there is a need to emit the light with various wavelengths, then multiple different LED diodes can be used within a single activation light, which are turned on either selectively or at the same time. It proved preferable to use a spectrum of the wavelength within a range from 700 to 1850 nm.

Proposed invention can be used during the designing of new welding technologies, but it will be very advantageously applicable for the existing welding devices. The main advantage lies in the fact that it is not necessary to modify the protective device itself in any way. The system can include a switch of the activation light which is designed for cooperation with the common trigger of the welding gun. The switch can have a form of a micro-switch, whereby the trigger of the welding gun is pressed through it. During the pressing of the trigger a switch is pressed, too. In order to achieve high universality of the device with the activation light, the activation light can be a part of the ring which is designed for mounting and attaching through the mouth of the welding torch. The ring is attached to the body of the torch or to the body of the gun, respectively. The ring carries at least one source of the activation light, preferably infrared LED diode. For the distribution of the luminous radiation to the other parts of the ring it is advantageous to use an optical diffuser. An arrangement can be used which has multiple LED diodes with different or identical light emitting characteristics - that is, mainly with different or identical wavelength. In case of multiple LED diodes these can be distributed around within a ring in order to secure reliable functioning of the activation light at various positions of the welding torch or welding gun.

Activation light can be in preferable arrangement covered by the replaceable transparent protective cover which is resistant to heat, mechanical stress and sparks which arise during the technological process, mainly during welding. Such cover will be designed as an easily replaceable expendable material which after the depreciation can be replaced for a new piece without tools. This protects the surface of the activation light, which for example in case of the LED light can be produced from plastic. The protective cover of the activation light can be circle-shaped or U-shaped and it can be produced from polycarbonate or glass.

Activation light or control electronics of the activation light, respectively, can have - besides the switch - a control circuit which sets its characteristics. It can set the delay against the beginning of the shining until the moment of the pressing of the switch. The frequency of the power supply can be also adjusted. It will be preferable if the period of the shining after which the activation light stops shining is adjusted, too. Activation light can also have an indication of the low battery or other service functions. In order to increase the longevity of the battery, the activation light can have a setting of the power supply.

In case of the implementation of the system according to this invention to the welding gun its trigger switch can be used, which is connected to the control electronics of the activation light. The starting switch in case of the welding guns for MIG/MAG, TIG is usually a micro-switch with high longevity of the switch cycles. It is preferable if a galvanic section - for example by means of relay or optocoupler - is produced for the switch after the control electronics is connected to it. The use of one switch for the launch of the welding process and control of the activation light can also have inverse hierarchy, where the switch of the control electronics is used to launch the welding process. This would mean that in case of the faulty switch of the control electronics - for example, in case of the empty battery of the activation light - the welding itself does not start, too. This improves the protection of the sight, since welding will be possible only with functioning system according to this invention.

Switch on the welding gun can be produced as two-stroke, when the switching always takes place consecutively. This can be advantageously used in a case where the zero pre-blow time of the protective gas is set and the electric arc is ignited just after the pressing of the switch. With two-stroke switch the first switch circuit controls control electronics of the activation light and second switch circuit starts the process of welding itself. Thanks to this there is a sufficient lead-time even in case of low or zero pre-blow of the protective gas.

It will be also advantageous if the activation light can be turned on for the testing purposes even without launching the technological process. Such regime serves for a simple test whether the given protective device reacts correctly to the activation light. In case the switch of the activation light itself is connected to the trigger of the welding gun it suffices that the trigger - and thereby the switch of the activation light - is pressed with the technological device turned off (for example, with the welding machine or welding source turned off). In case the switch of the activation light is included in the technological device, or in the case the switch is in software form, the independent hardware or software testing switch for initiation of the activation light can be produced in similar way.

Activation light together with the control electronics can be placed in the common body, on the common PCB board, which will be advantageous especially in case of arrangement which will be intended to supply the welding gun itself. The arrangement for independent supply of the existing welding guns will be compact. In case of designing a new welding gun or in case of a hardware modification of the welding gun an arrangement will be preferable where the control electronics and the activation light are in the separate parts. This ensures better mechanical, radiation, electromagnetic and heat protection of the control electronics, which can be placed in the body of the welding gun, for example in the handle of the welding gun. A small opening will suffice for the activation light, it can be produced in the dividing plane of the molding of the gun, which simplifies the given modification of the injection mold.

Activation light placed in the welding gun can be placed on the surface of its outer body or it can be placed on its inside and the light flow from the activation light will be led by means of the optical conductor to the surface of the welding gun, which improves the protection of the activation light. In this sense there are multiple versions and combinations with varying number of the sources of the activation light possible, with various arrangements of the conduction of the optical signal to the surface of the welding gun.

The placement of the control electronics inside the body of the welding gun offers good spatial possibilities of casing and it offers sufficient space for the power source, for example an accumulator or a removable battery, which can be approachable in the opening shaft. In order to achieve larger building space without increasing the weight of the welding gun held by the welder, the control electronics can be placed outside the welding gun - for example in the connecting ending or connector by which the hose with the protective gas and a cable or wire with the welding current are connected to the welding machine. It can be, for example, euro-connector which in standardized realization is used by multiple producers of the welding machines and welding sources. Electric conductors then reach out of this place, out of the control electronics, to the welding gun, where the activation light is placed, too. The location of the control electronics in or by the connector allows to create sufficiently large casing of the control electronics. With such arrangement the control electronics can supplied by power from its own adapter in the electric network, or the adapter is used to recharge the accumulator. The control electronics placed by or in the connecting ending greatly removes the risks relating to being in the vicinity of the welding process.

Placement of the activation light within or on the welding gun successfully covers most welding situations and most welding positions. Welder usually sees welding gun so that he or she can move it alongside the weld. There are, however, situations where the welder works in an environment where the optical sensor of the protective device is covered (for example during welding of the metal frames). In such case the darkening of the protective device (helmet) may not take place at all, because even though the welder sees the electric arc by his or her own eyes, the optical sensor is covered by the environment (for example a flat piece of metal right before the helmet, crossing the field of view of the optical sensor) and the optical information causing the darkening of the protective device does not reach it.

This is a dangerous phenomenon because not only the delay of the darkening of the protective device, which is eliminated according to this invention, will not take place - there will be no darkening even during the luminous manifestation. Here the proposed invention offers advantage of the independent transfer of the information concerning the luminous manifestation. One of the sources of the activation light can be produced as external, which is connected to the control electronics by the flexible cable or attached onto the sleeve, glove or other appropriate place. In order to attach external source of the activation light a Velcro fastener or a clip or another appropriate mechanical mean can be used, or the clothing or glove can be designed for this purpose, for example sewed on as a small, transparent socket which also constitutes a protective cover. Externally attachable activation light can be a single one or only an additional activation light in a system with multiple sources of activation lights. A connector (for example jack plug) can be used on the surface of the welding gun, where the cable of the external source of the activation light is connected into. The connection can - pursuant to the setting - cause disconnection of the stable activation light or both sources can shine simultaneously. External source of the activation light can be connected on the body of the welder closer to the optical sensor or directly on the protective device, for example on the helmet.

The disclosed principle of the activation of the optical element with the changing permeability can be used to increase the sensitivity of inferior or not sensitive enough protective devices which do not protect the sight of a man sufficiently and which recognize the luminous manifestation unreliably or only at higher intensity. In order to improve the transfer of the optical information from the activation light one can use an element to repeat the signal which detects on its input the turning on of the activation light and on its output it further turns on its own activation light. Such element for repetition of the signal works as its spatial diffuser, extender, or amplifier. It can be used in situation where the optical sensor on the protective device is shielded or in a situation where the optical sensor is not sensitive enough. The element for repetition of the signal can be attached in the close proximity of the optical sensor; that is, for example, directly on the welding helmet. Activation light of the element for the repetition of the signal will be placed in the proximity of the optical sensor.

To supply the control electronics with power a one-off removable battery can be used; the energy demands of the system are low. Rechargeable accumulator can be used, too, which is replaceable or it is charged by the adapter or it is charged by the solar cell as the welding helmet does, too. A solution appears preferable where the accumulator is charged by the induction of the flowing current in the welding cable. The proximity of the welding cable and hose with the flowing protective gas can be used for the purpose of charging by means of dynamo with a little propeller, which uses the energy of the flowing protective gas. Another possibility is the supply by the stable source from the electric network by the respective adapter. Such connection is advantageous mainly when the control electronics of the activation light is placed outside the welding gun, for example in the connector of the cable and the hose.

The protection devices included in the system according to this invention are capable of realizing its function even without the system, whereby all original features are maintained. The method and system according to this invention do not require any preparation or adjustment of the protective devices; these can be connected to the system on the basis of the original optical sensor, whereby we emulate the signal from the luminous manifestation (in a way which is not harmful to health) before the creation of the actual luminous manifestation.

Important advantage of the proposed solution is precisely its universal use in relation with any protective device which has an optical sensor for the detection of the luminous manifestation of the technological process. The solution can be used with the existing welding helmets, masks, shields or goggles. The technological process can be manual, automatized or combined. Usually it will be arc or laser welding or cutting or plasma cutting and so on. Use during arc welding with the protective gas will be advantageous. The method and system does not require modification or completion of the protective device. Even protective devices with the slow reaction - that is, cheap protective devices, too - can with this solution achieve very short, basically zero or negative reaction time against the point in time when the luminous manifestation of the technological process appears. This allows to lower overall costs for the protection of sight and to improve the health of the welders.

### Brief description of drawings

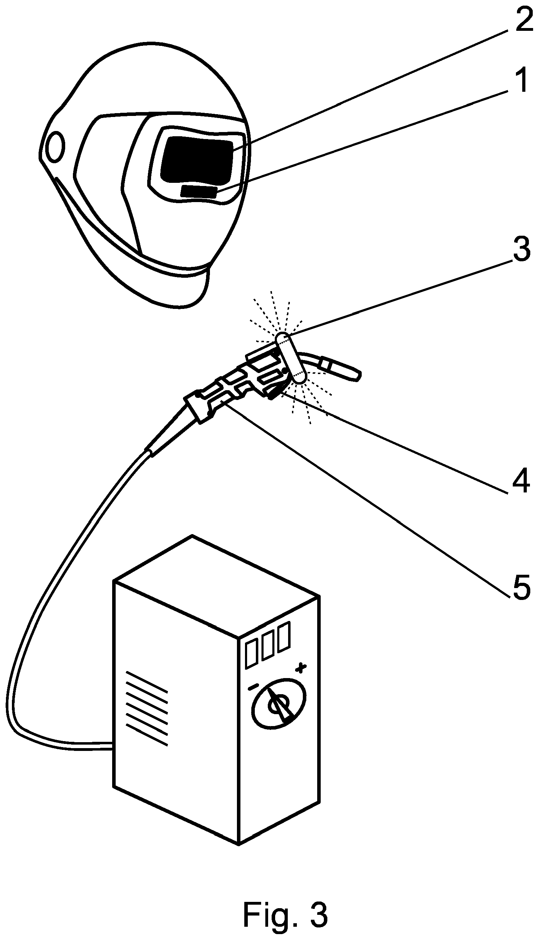

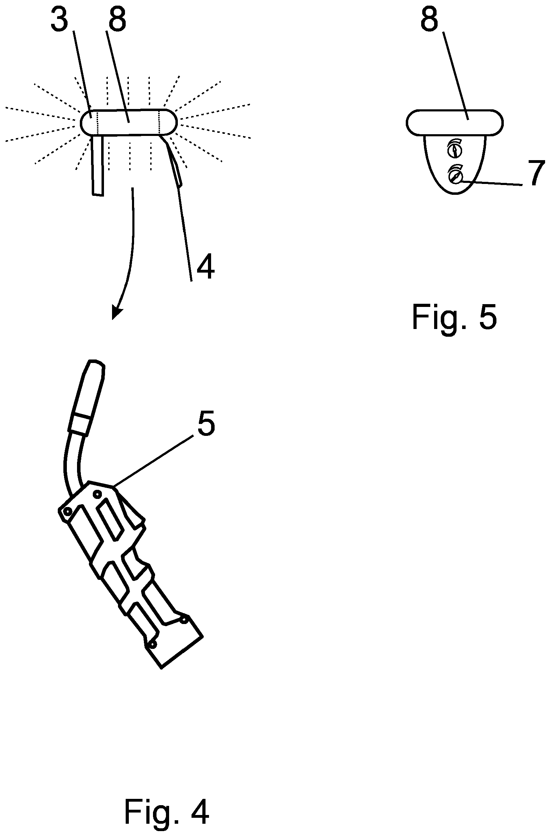

The invention is further disclosed by the figures 1 to 18. The used scales and ratios between the individual elements of the system, the shape of the activation light as well as described temporal ratios are not binding, they are informative or they have been adjusted in order to increase clarity.
Figure 1 depicts a time graph (timing chart) with the course of the welding and darkening of the welding helmet pursuant to the state of the art. Process G corresponds to the inflow of the protective gas and it begins with the pressing of the trigger on the welding gun. Process E is an ignition and burning of the electric arc. Process D is darkening of the welding helmet. Time period x denotes a delay of the darkening of the protective device relative to the luminous manifestation.
Figure 2 is a time graph (timing chart) with the course of the welding and darkening of the welding helmet pursuant to this invention. Process A is shining of the activation light. Phase I on the figures 1 and 2 is pre-gas phase, phase II is a period of burning electric arc, phase III on figures 1 and 2 is post-gas phase. Time a is intentional delay of the shining of the activation light against the instruction to start the technological process. Time b is lead-time of the activation light before the luminous manifestation. Time c is a lead-time of the darkening before the appearance of luminous manifestation.
Figure 3 is an axonometric view of the workplace of the welder with the manually controlled welding gun on which there is a ring-shaped activation light. The interrupted lines stemming from the activation light denote infrared radiation.
Figure 4 depicts activation light. The arrow denotes the direction in which the ring with the activation light is put on the welding gun. The interrupted lines stemming from the activation line denote infrared radiation which is spreading around the activation light. Figure 5 show activation light from the figure 4 rotated in such a way that its body with the control elements can be seen.

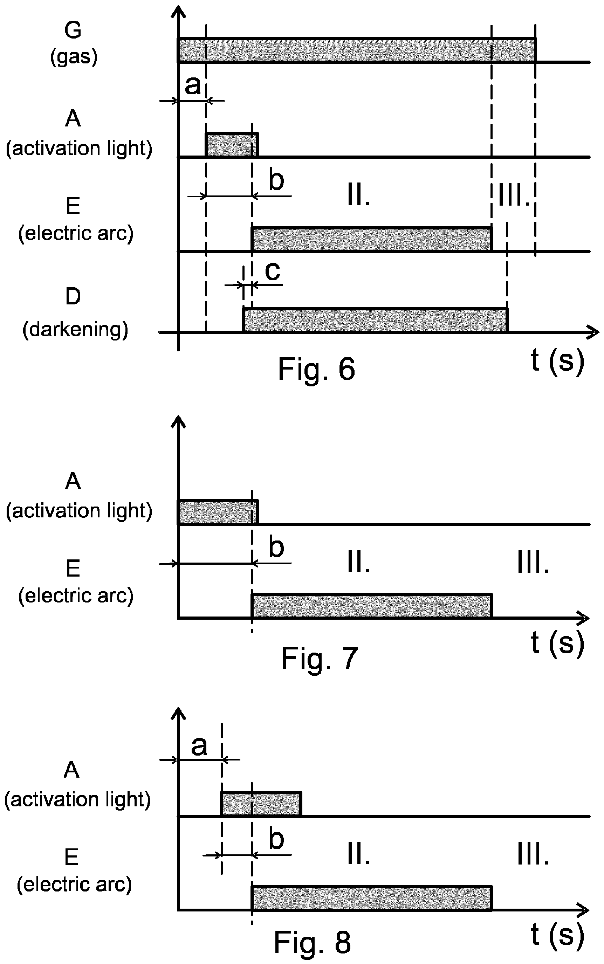

Figure 6 is a time graph (timing chart) with the course of the welding and darkening of the protective device with the turning off of the activation light after the detection of the luminous manifestation. The time of the activation shining is shorter than in the case of figure 2 where the activation light shines for a set period. Time a is intentional delay of the shining of the activation light relative to the instruction to start the technological process. Time b is a lead-time of the activation light before the luminous manifestation. Time c is a lead-time of the darkening before the appearance of the luminous manifestation.
Figures 7 and 8 show the steps of adaptation of the control of the activation light. In the first step according to figure 7 the activation light starts to shine right after the instruction to initiate the technological process. In the x-th step - for example in the second step of the adaptation according to figure 8 - the control has set the delay "a", in another step this can be shortened. The period of shining of the activation light is on the figures 7 and 8 depicted to be the same in order to increase clarity, but this adaptation can be with the turning off of the activation light as depicted on the figure 6.
Figure 9 is an algorithm of the method during darkening of the welding helmet pursuant to the state of the art.
Subsequently, figure 10 is a method during darkening of the protective device with use of the activation light.
Figure 11 is a block diagram of the basic elements of the control of the activation light. Figure 12 is a block diagram with the turning off of the activation light according to the instruction from the second optical sensor.
Figure 13 depicts an adaptation of the control of the activation light during the technological process where there is a first series of cycles after the first pressing of the trigger. Activation light is turned on during the first pressing of the trigger and then for two cycles the protective device is darkened only on the basis of the detection of the luminous manifestation itself.
Figure 14 depicts the placement of the control electronics inside the body of the welding gun.
Figure 15 depicts the placement of the control electronics in the euro-connector of the hose and the cable.
Figure 16 depicts the independent placement of the control electronics supplied by power from AC/DC adapter from the electric network.
Figure 17 is a schematic connection of the external activation light, and it is also suggested that the switch of the control electronics is a trigger of the welding gun.
Figure 18 depicts an element to repeat the signal which carries a signal from the activation light to the proximity of the optical sensor on the welding helmet.

### Examples of realization

### Example 1

System according to this example on the drawings 2, 3, 4, 5, 10 and 11 is used in a common workshop without systematic integration. The welding apparatus for the MIG/MAG welding by the melting electrode in the protective atmosphere has a welding gun 5 with the trigger which starts the technological process - the inflow of the gas, the movement of the wire and the electric arc. The welder has self-darkening helmet which is produced by a different producer than the welding device. During the welding the welding helmet reacts to the detected luminous manifestation of the welding. The welding helmet in this example has a reaction time of the darkening at the level of 0,15 ms. During this period the sight of the welder is exposed to the effects of the dangerous radiation.

The set in this example is supplied by the activation light 3. This is part of the small ring which inner diameter is larger than the diameter of the torch on the welding gun 5. The ring has removable inner ringlets - their gradual removal or addition can set the desired inner diameter of the ring. There are two infrared LED diodes placed on the opposite sides inside the ring. Both are covered by the optical diffuser 8 which distributes the emitted light to the environment.

Both infrared LED diodes have wavelength 850 nm, approximate angular spread of 140° and they are supplied by power from common source which has a frequency excitation on its output. The value of frequency on the frequency generator 6 is adjustable by means of a small rotary potentiometer ranging from 5 to 250 Hz. Similarly, the period of the delay of the beginning of the activation light from the moment of the switch 4 is adjustable, too. This period can be set from 0 to 1 second with help of adjusting element 7 of the delay. The third adjusting element serves to set the period of the activation shining. In this example the period can be set from 1 to 3 seconds. Fourth adjusting element changes the power supply of the infrared LED diodes; in common conditions the power supply of 250 mW suffices for the activation of one LED diode. The harmlessness of the activation light 3 for the unprotected sight of the surrounding personnel is proved by the fact that some sort of LED diodes is used in remote controllers of the home electronics. The use of the infrared remote controller is not considered a risk even in situation where the invisible radiation is directed straight to a human eye.

Switch 4 of the activation light 3 has in this example form of the flat switch circuit which is glued to the controlling edge of the trigger. The pressing of the trigger causes, firstly, pressing of the switch 4 and then turning on of the trigger of the welding apparatus, too.

After the pressing of the trigger of the welding gun 5 the welding apparatus opens a valve with the protective gas. Approximately 0,5 s after the opening of the valve the electric arc is turned on which starts the technological process. Simultaneously with the pressing of the trigger the switch 4 of the activation light 3 has been pressed. The control circuit delays the turning on of the activation light 3 by approximately 0,4 s since the pressing of the switch 4. At that moment the activation light 3 shines and the optical sensor 1 on the welding helmet reacts in such a way that it issues an instruction to darken the optical element 2. This instruction enters the optical element 2 approximately 0,1 s before the ignition of the electric arc itself. At time 0,5 s, when the luminous manifestation of the welding appears, the welding helmet is already darkened. This basically achieves negative darkening time of the welding helmet at approximately 0,1 s.

### Example 2

Automatic welding line in the body car works have multiple activation lights 3 distributed on the fixed spots. The control system of the welding issues an instruction to initiate the activation light 3 approx. 0,1 second before the instruction to ignite the electric arc in a give section of the line. Workers who oversee and check the process watch the line through the welding helmet with the self-darkening optical element 2. This reacts to the instruction from the optical sensor 1 which detects, firstly, the activation light and then the luminous manifestation of the technological process itself.

### Example 3

System in the example is used with the laser cutting machine. The switch 4 has a software form. Before bringing the laser beam to the place of cutting the instruction is issued by central controlling system to light up the activation lights 3 placed on the working arm as well the edges of the table. One activation light 3 can be directed either directly; others can form parts of the independent elements 11 to repeat the signal.

### Example 4

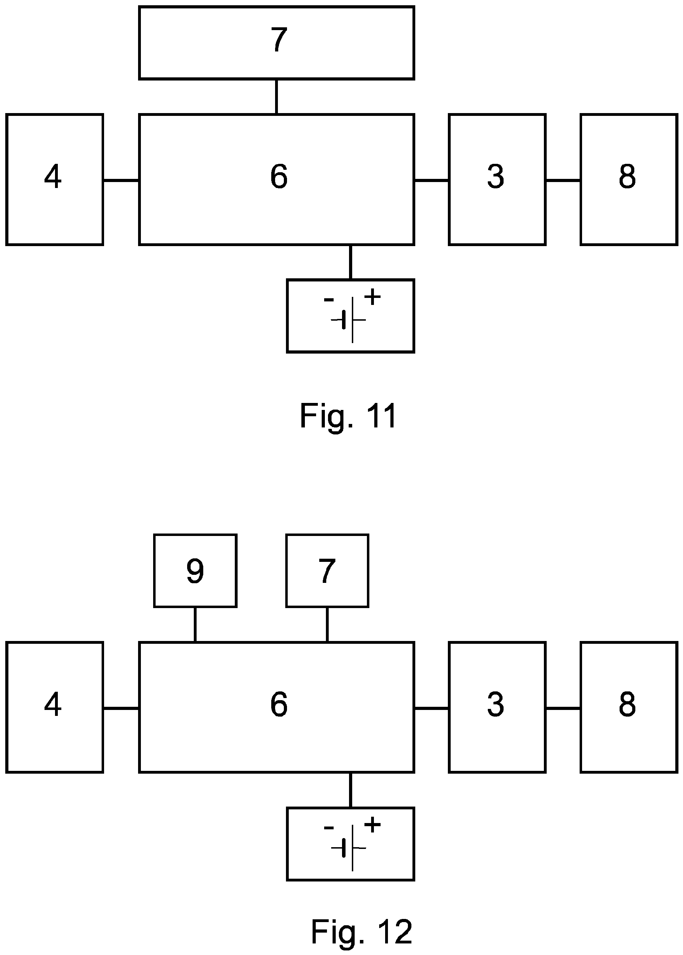

Activation light 3 according to figures 6 and 12 has its own control of the turning off. This function works on the basis of the cooperation with the second optical sensor 9 which in this example is placed in the ring on the welding gun 5. The control of the activation light 3 receives an information that the second optical sensor 9 recorded the luminous manifestation of the technological process. This information means that the activation light is no longer needed and on the basis of this information the activation light 3 is turned off.

Since the second optical sensor 9 can be faster than the optical sensor 1 of the unknown producer of the protective device, the instruction to turn off the activation light 3 can be intentionally delayed by e.g. 3 ms, when the common or inferior protective devices already react to the luminous manifestation.

### Example 5

Control of the activation light 3 involves an adaptation algorithm which measures a period between the instruction to initiate the technological process and the appearance of the luminous manifestation. Pursuant to the measured time period according to figures 7 and 8 the delay of the lighting up of the activation light 3 is in the iterative steps gradually increased until the moment where the lead-time of the lighting up of the activation light 3 against the luminous manifestation is at 0,1 s. This delay is then used for the following strokes, whereby it is continually measured whether there is sufficient lead-time of lighting up of the activation light 3 before the luminous manifestation.

### Example 6

Activation light 3 is turned on at the first pressing of the trigger, then during two cycles the protective device is darkened only on the basis of the detection of the luminous manifestation itself because the cycles are not accompanied by the pressing of the switch 4. After three cycles the control of the activation light 3 adapts to the detected course of the luminous manifestation and before the expected fourth and each following luminous manifestation the control lights up the activation light 3. The consequence of this approach is that the second and third darkening of the protective device is delayed according to the reaction time of the given protective device; the next darkening is realized with the lead-time.

### Example 7

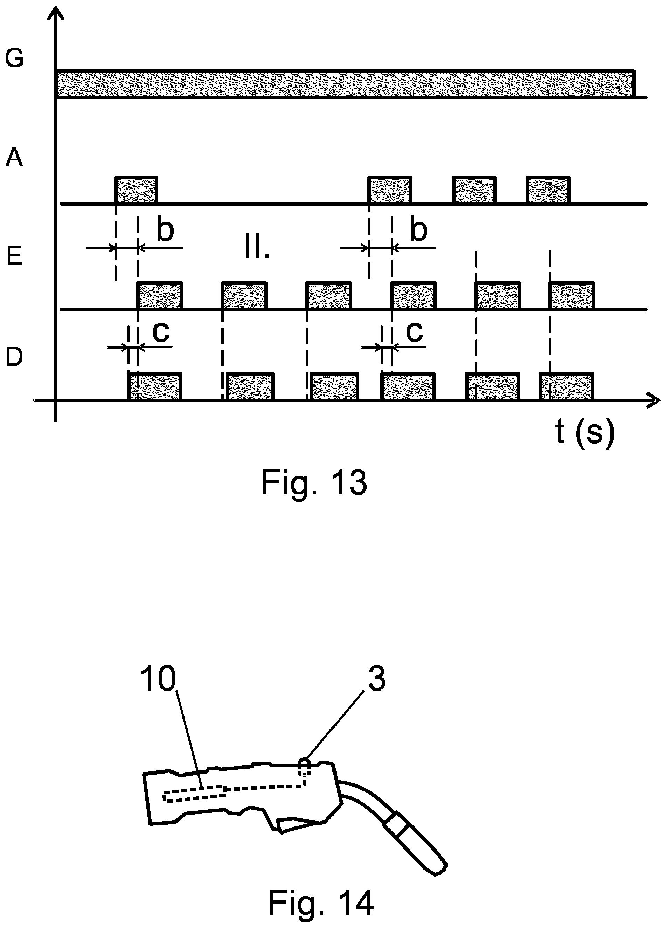

Activation light 3 according to the figure 14 is cased in the plastic body of the welding gun 5. On the surface of the welding gun 5 there is an opening through which the removable cover of the LED diode of the activation light 3 protrudes. The cover is produced from the transparent plastic and in case of damage it can be pulled out by rotating and changed.

Control electronics 10 are placed in the handle of the welding gun 5 where the opening shaft for the insertion of the battery or the rechargeable accumulator is produced.

### Example 8

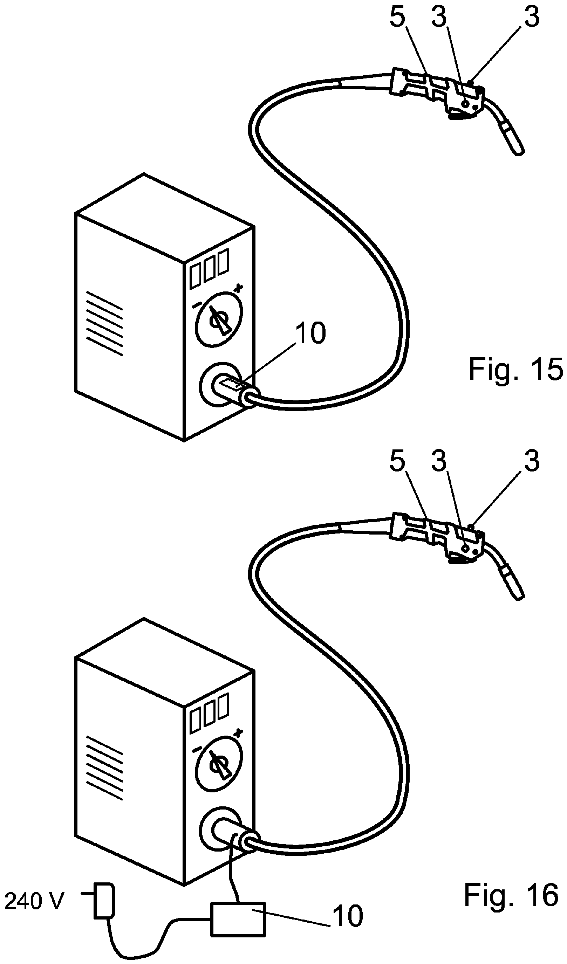

Activation light 3 with three sources in form of the LED diodes according to figure 15 is placed in the plastic body of the welding gun 5 in the similar way as in the previous example. Control electronics 10 are placed in the connector of the hose and the electric cable - in this example it is a standardized euro-connector.

### Example 9

Control electronics 10 are in the independent box which is close to the welding machine, whereby the connecting cable connecting the control electronics 10 with the activation light 3 is connected to the hose with the protective gas. In order to supply the control electronics 10 and the activation light 3 with power an ACD/CD adapter is used, in this example with microUSB ending; the connector on the body of the control electronics 10 corresponds to it. The use of the USB power supplying adapter with 5V voltage simplifies the construction of the system.

### Example 10

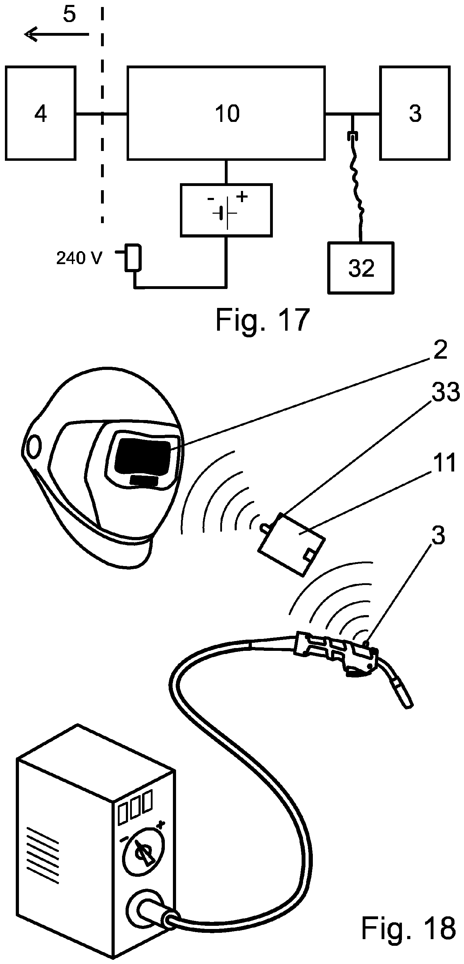

In this example according to the figure 17 the trigger of the welding gun 5 itself is used as switch 4. On the surface of the welding gun 5 there is a jack connector which allows for connection of the external activation light 32. This has a form of the LED diode in the casing with the mechanical clip in order to attach it to the edge of the glove or sleeve of the protective clothing of the welder. In this example the external activation light 32 shines together with the activation light 3 on the welding gun 5.

### Example 11

System in this example according to the figure 18 is supplied by the independent signal repeating element 11. It has an outer form of the small box with its own source of the electric energy. Signal repeating element 11 has a receiver of the optical signal and the transmitter in form of the signal repeating activation light 33. After receiving the light from the activation light 3 the signal repeating activation light 33 - which can be attached closer to the optical sensor 1 of the welding helmet - lights up. Signal repeating element 11 can be equipped by the adhesive layer in such a way that signal repeating activation light can be directly glued within the field of view of the optical sensor 1 of the welding helmet.

Following the above mentioned features and methods the person skilled in the art can - even without inventive activity - produce other arrangements and methods which use the main features of this invention.

### Industrial applicability

Industrial applicability is obvious. According to this invention it is possible to repeatedly produce and use systems for the initiation of the darkening of the optical element in advance before the initiation of the luminous manifestation

### List of related symbols

- 1-: optical sensor
- 2-: optical element with changeable permeability
- 3-: activation light
- 32 -: external activation light
- 33 -: signal repeating activation light
- 4-: switch
- 5-: welding gun
- 6-: frequency generator
- 7-: adjusting element of delay
- 8-: optical diffuser
- 9-: second optical sensor
- 10-: control electronics
- 11-: signal repeating element

- MIG -: Metal Inert Gas
- MAG -: Metal Active Gas
- TIG, WIG -: tungsten (wolfram) inert gas welding
- PCB -: printed circuit board
- 240 V -: electrical distribution network with intermittent voltage
- ADF -: automatic darkening filters

## Claims

1. A system for an accelerated reaction of a darkening of an optical element in a welding personal protective device for a protection of a sight which includes an optical sensor (1), an aperture with an optical element (2) with an adjustable permeability for a limitation of a permeation of a radiation from a luminous manifestation to the sight, where the optical sensor (1) for a detection of the luminous manifestation of an electric arc welding is placed within the welding personal protective device, and the optical sensor (1) is connected with a control of the optical element (2), it includes an activation light (3) emitting radiation which is detected by the optical sensor (1), whereby the activation light (3) is placed within a reach of the optical sensor (1),
is **characterized by the fact**, that
this optical sensor (1) is the same sensor which is used within the welding personal protective device to detect the luminous manifestation of the electric arc welding itself, where the activation light (3) is controlled by a switch (4) which is connected with the activation of the electric arc welding in such a way that the switch (4) is activated before a transmission of an electric current between an electrode and a welded material,

2. The system for the accelerated reaction of the darkening of the optical element in the welding personal protective device according to the claim 1 is **characterized by the fact**, that the activation light (3) has at least one infrared LED diode, preferably it includes multiple LED diodes with different emitting characteristics.

3. The system for the accelerated reaction of the darkening of the optical element in the welding personal protective device according to the claim 1 or 2 is **characterized by the fact**, that the activation light (3) is a part of a body which is adjusted for a connection to a welding gun (5), preferably the body carries adjusting elements, a power supply and a switch (4), where the body is ring-shaped and the activation light (3) has an optical diffuser (8).

4. The system for the accelerated reaction of the darkening of the optical element in the welding personal protective device according to the claim 1 or 2 is **characterized by the fact**, that the activation light (3) is a part of a body of the welding gun (5), and the switch (4) is a switch of the welding gun (5); preferably the switch (4) is a two-phase switch.

5. The system for the accelerated reaction of the darkening of the optical element in the welding personal protective device according to any of the claims 1 to 4 is **characterized by the fact**, that it includes a generator (6) of a frequency of the power supply of the activation light (3), preferably the generator (6) of the frequency is adjustable; it also includes an adjustable element (7) of a delay of the activation light (3) relative to the switching of the switch (4) and an element for an adjustment of a period of shining of the activation light (3).

6. The system for the accelerated reaction of the darkening of the optical element in the welding personal protective device according to any of the claims 1 to 5 **is characterized by the fact**, that it includes an element for the detection of the luminous manifestation which is connected to the control of the activation light (3) and preferably it includes second optical sensor (9), too, whereby the activation light (3) has a block for turning off of the activation light (3) after a creation of the luminous manifestation or it has a block for adaptation of the delay of the lighting up of the activation light (3) relative to the switching of the switch (4) or a block for skipping the lighting up of the activation light (3) in every second switching of the switch (4) or a block for lighting up the activation light (3) in cycles calculated on a basis of a previous course of a technological process.

7. The system for the accelerated reaction of the darkening of the optical element in the welding personal protective device according to any of the claims 1, 2, 4 to 6 **is characterized by the fact**, that controlling electronics (10) of the activation light (3) are part of the body of the welding gun (5) or it is a part of a connecting end of a tube and electric cables which connect the welding gun (5) with a welding source, whereby the controlling electronics (10) are connected with the activation light (3) by means of electric conductors which are at least in part of their length connected to a bunch of the tube and the electric cables.

8. The system for the accelerated reaction of the darkening of the optical element in the welding personal protective device according to any of the claims 1 to 7 **is characterized by the fact**, that it has at least one external activation light (32) which is connected with the controlling electronics (10) by a flexible cable; preferably the external activation light (32) has a connecting element for its placement on a clothing of a personnel.

9. The system for the accelerated reaction of the darkening of the optical element in the welding personal protective device according to any of the claims 1 to 8 **is characterized by the fact**, that in includes a signal repeating element (11) which has a receiver of the optical signal of the activation light (3) and it has a transmitter in form of an activation light (33) to repeat the signal, whereby the signal repeating element (11) is adjusted for an independent placement in a vicinity of the optical sensor (1).

10. A method for an accelerated reaction of a darkening of an optical element in a welding personal protective device for a protection of a sight during an electric arc welding, with use of the system according to any of the claims 1 to 9, where a luminous manifestation arises due to a transmission of an electric current between an electrode and a welded material, whereby this luminous manifestation is detected by an optical sensor (1) on the welding personal protective device, and where on a basis of a detected signal from the optical sensor (1) the darkening of the optical element (2) is activated, where this optical element (2) by its darkening limits a penetration of a radiation from the luminous manifestation to the sight whereby an instruction to begin the welding is detected inside the device which realizes the electric arc welding before the transmission of the electric current between the electrode and the welded material;
an activation light (3), which shines in a field of a vision of the optical sensor (1) on the welding personal protective device, is turned on on a basis of this instruction, which causes the activation of optical sensor (1) by means of the activation light (3) before the optical sensor (1) detects the respective luminous manifestation of the electric arc welding;
whereby the activation light (3) shines at least until a moment when the optical sensor (1) of the welding personal protective device itself detects the luminous manifestation of the electric arc welding itself.

11. The method for the accelerated reaction of the darkening of the optical element in the welding personal protective device according to the claim 10 **is characterized by the fact**, that after the detection of the luminous manifestation by the respective optical sensor (1) on the welding personal protective device the activation light (3) is turned off by the system.

12. The method for the accelerated reaction of the darkening of the optical element in the welding personal protective device according to the claim 10 or 11 **is characterized by the fact**, that the instruction to turn on the activation light (3) during the electric arc welding in a protective atmosphere is issued during a pre-gas time, where a protective gas is transferred to a welding point before an ignition of the electric arc.

13. The method for the accelerated reaction of the darkening of the optical element in the welding personal protective device according to the claim 10 or 12 **is characterized by the fact**, that the activation light (3) shines for a preset time since it lights up, preferably for at least 1 second, or the activation light (3) goes off after its control receives information about the detection of the luminous manifestation, preferably it receives such information from the second optical sensor (9).

14. The method for the accelerated reaction of the darkening of the optical element in the welding personal protective device according to any of the claims 10 to 13 **is characterized by the fact**, that the activation light (3) lights up 0,05 to 0,5 second before the creation of the luminous manifestation, preferably it lights up with the delay up to 0,5 s against the instruction to ignite the electric arc.

15. The method for the accelerated reaction of the darkening of the optical element in the welding personal protective device according to any of the claims 10 to 14 **is characterized by the fact**, that the control of activation light (3) calculates a time between the lighting up of the activation light (3) and the luminous manifestation of the electric arc welding and it adjusts the delay of the lighting up of the activation light (3) on the basis of a calculated value in such a way that in a next step of welding the desired delay is achieved, preferably the delay will be 0,1 s at maximum.

16. The method for the accelerated reaction of the darkening of the optical element in the welding personal protective device according to any of the claims 10 to 15 **is characterized by the fact**, that the activation light (3) emits the radiation with a wavelength between 700 and 1850 nm and with the frequency ranging from 5 to 250 Hz.

## Patentansprüche

1. System für eine beschleunigte Verdunkelungsreaktion eines optischen Elements an einer persönlichen Schweißschutzvorrichtung zum Sichtschutz, welche einen optischen Sensor (1) und eine Öffnung mit einem optischen Element (2) mit regulierbarer Durchlässigkeit zur Begrenzung des Strahlungsdurchgangs von einer Lichtmanifestation zu den Augen umfasst, wobei der optische Sensor (1) zur Erfassung der Lichtmanifestation eines Lichtbogenschweißens an der persönlichen Schweißschutzvorrichtung positioniert ist und der optische Sensor (1) mit einer Steuerung des optischen Elements (2) verbunden ist, ein Aktivierungslicht (3) umfassend, das eine Strahlung abgibt, die durch den optischen Sensor (1) erfasst wird, wobei das Aktivierungslicht (3) innerhalb der Reichweite des optischen Sensors (1) positioniert ist,
**dadurch gekennzeichnet, dass** dieser optische Sensor (1) derselbe Sensor ist, der innerhalb der persönlichen Schweißschutzvorrichtung dazu verwendet wird, die Lichtmanifestation des Lichtbogenschweißens selbst zu erfassen,
wobei das Aktivierungslicht (3) durch einen Schalter (4) gesteuert wird, der derart mit der Aktivierung des Lichtbogenschweißens verbunden ist, dass der Schalter (4) aktiviert wird, bevor zwischen einer Elektrode und einem geschweißten Materialelektrischer Strom übertragen wird.

2. System für die beschleunigte Verdunkelungsreaktion des optischen Elements an der persönlichen Schweißschutzvorrichtung nach Patentanspruch 1, **dadurch gekennzeichnet, dass** das Aktivierungslicht (3) wenigstens eine Infrarot-LED-Diode aufweist und vorzugsweise Mehrfach-EED-Dioden mit unterschiedlichen Emissionsmerkmalen umfasst.

3. System für die beschleunigte Verdunkelungsreaktion des optischen Elements an der persönlichen Schweißschutzvorrichtung nach Patentanspruch 1 oder 2, **dadurch gekennzeichnet, dass** das Aktivierungslicht (3) Teil eines Körpers ist, der zur Verbindung an eine Schweißpistole (5) ausgelegt ist, wobei der Körper vorzugsweise Einstellelemente, eine Stromversorgung und einen Schalter (4) umfasst, wobei der Körper ringförmig ist und das Aktivierungslicht (3) einen optischen Diffusor (8) aufweist.

4. System für die beschleunigte Verdunkelungsreaktion des optischen Elements an der persönlichen Schweißschutzvorrichtung nach Patentanspruch 1 oder 2, **dadurch gekennzeichnet, dass** das Aktivierungslicht (3) Teil des Körpers der Schweißpistole (5) ist, und der Schalter (4) ein Schalter der Schweißpistole (5) ist, und der Schalter (4) vorzugsweise ein Zweiphasenschalter ist.

5. System für die beschleunigte Verdunkelungsreaktion des optischen Elements an der persönlichen Schweißschutzvorrichtung nacheinem jeden der Patentansprüche 1 bis 4, **dadurch gekennzeichnet, dass** es einen Generator (6) einer Frequenz der Stromversorgung des Aktivierungslichts (3) umfasst, wobei der Frequenzgenerator (6) vorzugsweise verstellbar ist, und des Weiteren ein verstellbares Element (7) für die Verzögerung des Aktivierungslichts (3) bezüglich des Schaltens des Schalters (4) umfassend, sowie ein Element zur Einstellung der Zeit, in der das Aktivierungslicht (3) scheint.

6. System für die beschleunigte Verdunkelungsreaktion des optischen Elements an der persönlichen Schweißschutzvorrichtung nach einem jeden der Patentansprüche 1 bis 5, **dadurch gekennzeichnet, dass** es ein Element zur Erfassung der Lichtmanifestation umfasst, das mit der Steuerung des Aktivierungslichts (3) verbunden ist und vorzugsweise auch einen zweiten optischen Sensor (9) umfasst, wobei das Aktivierungslicht (3) einen Block für die Abschaltung des Aktivierungslichts (3) nach der Erzeugung der Lichtmanifestation aufweist oder einen Block für die Anpassung der Verzögerung des Aufleuchtens des Aktivierungslichts (3) bezüglich des Schaltens des Schalters (4) oder einen Block für das Auslassen des Aufleuchtens des Aktivierungslichts (3) bei jedem zweiten Schalten des Schalters (4) oder einen Block für das Aufleuchten des Aktivierungslichts (3) in Zyklen, die auf Basis eines vorhergehenden Verlaufs eines technologischen Verfahrens berechnet wurden.

7. System für die beschleunigte Verdunkelungsreaktion des optischen Elements an der persönlichen Schweißschutzvorrichtung nach einem jeden der Patentansprüche 1, 2, 4 bis 6, **dadurch gekennzeichnet, dass** Steuerelektronik (10) des Aktivierungslichts (3) Teil des Körpers der Schweißpistole (5) ist oder Teil eines Verbindungsendes einer Röhre mit Stromkabeln ist, welche die Schweißpistole (5) mit einer Schweißquelle verbinden, wobei die Steuerelektronik (10) mit dem Aktivierungslicht (3) verbunden ist durch elektrische Leiter, die wenigstens über einen Teil ihrer Länge mit einem Bündel der Röhre und den Stromkabeln verbunden sind.

8. System für die beschleunigte Verdunkelungsreaktion des optischen Elements an der persönlichen Schweißschutzvorrichtung nach einem jeden der Patentansprüche 1 bis 7, **dadurch gekennzeichnet, dass** es wenigstens ein externes Aktivierungslicht (32) umfasst, das über ein flexibles Kabel mit der Steuerelektronik (10) verbunden ist, wobei das externe Aktivierungslicht (32) vorzugsweise ein Verbindungselement für seine Positionierung an der Kleidung eines Bedieners aufweist.

9. System für die beschleunigte Verdunkelungsreaktion des optischen Elements an der persönlichen Schweißschutzvorrichtung nach einem jeden der Patentansprüche 1 bis 8, **dadurch gekennzeichnet, dass** es ein Signalwiederholungselement (11) umfasst, das einen Empfänger für das optische Signal des Aktivierungslichts (3) sowie einen Sender in Form eines Aktivierungslichts (33) zur Wiederholung des Signals aufweist, wobei das Signalwiederholungselement (11) für die unabhängige Positionierung in der Nähe des optischen Sensors (1) eingestellt ist.

10. Verfahren für eine beschleunigte Verdunkelungsreaktion eines optischen Elements an einer persönlichen Schweißschutzvorrichtung zum Sichtschutz während des Lichtbogenschweißens unter Nutzung des Systems nach einem jeden der Patentansprüche 1 bis 9, wobei infolge der Übertragung von elektrischem Strom zwischen einer Elektrode und einem geschweißten Material eine Lichtmanifestation eintritt, wobei diese Lichtmanifestation durch einen optischen Sensor (1) an der persönlichen Schweißschutzvorrichtung erfasst wird, und wobei auf Basis eines durch den optischen Sensor (1) erfassten Signals die Verdunkelung des optischen Elements (2) aktiviert wird, wobei dieses optische Element (2) durch seine Verdunkelung den Durchgang einer Strahlung von der Lichtmanifestation zum Auge begrenzt,
wobei
vor der Übertragung des Stroms zwischen der Elektrode und dem geschweißten Material eine Anweisung zur Aufnahme des Schweißens innerhalb der Vorrichtung, die das Lichtbogenschweißen ausführt, erfasst wird;
wobei ein Aktivierungslicht (3), das in einem Sichtfeld des optischen Sensors (1) an der persönlichen Schweißschutzvorrichtung scheint, auf Basis einer Anweisung eingeschaltet wird, welche die Aktivierung des optischen Sensors (1) mittels des Aktivierungslichts (3) selbst bewirkt, bevor der optische Sensor (1) die entsprechende Lichtmanifestation des Lichtbogenschweißens erfasst;
wobei das Aktivierungslicht (3) wenigstens bis zu dem Moment scheint, in dem der optische Sensor (1) der persönlichen Schweißschutzvorrichtung selbst die Lichtmanifestation des Lichtbogenschweißens selbst erfasst.

11. Verfahren für die beschleunigte Verdunkelungsreaktion des optischen Elements an der persönlichen Schweißschutzvorrichtung nach Patentanspruch 10, **dadurch gekennzeichnet, dass** das Aktivierungslicht (3) nach der Erfassung der Lichtmanifestation durch den entsprechenden optischen Sensor (1) an der persönlichen Schweißschutzvorrichtung durch das System abgeschaltet wird.

12. Verfahren für die beschleunigte Verdunkelungsreaktion des optischen Elements an der persönlichen Schweißschutzvorrichtung nach Patentanspruch 10 oder 11, **dadurch gekennzeichnet, dass** die Anweisung zur Einschaltung des Aktivierungslichts (3) während des Lichtbogenschweißens in Schutzatmosphäre während einer Vorgaszeit abgegeben wird, in der vor der Zündung des Lichtbogens ein Schutzgas an einen Schweißpunkt übertragen wird.

13. Verfahren für die beschleunigte Verdunkelungsreaktion des optischen Elements an der persönlichen Schweißschutzvorrichtung nach Patentanspruch 10 oder 12, **dadurch gekennzeichnet, dass** das Aktivierungslicht (3) ab seinem Aufleuchten für eine Voreinstellzeit scheint, vorzugsweise wenigstens 1 Sekunde lang, oder dadurch, dass das Aktivierungslicht (3) erlischt, nachdem seine Steuerung die Information der Erfassung der Lichtmanifestation empfangen hat, und diese Information vorzugsweise von dem zweiten optischen Sensor (9) empfängt.

14. Verfahren für die beschleunigte Verdunkelungsreaktion des optischen Elements an der persönlichen Schweißschutzvorrichtung nach einem jeden der Patentansprüche 10 bis 13, **dadurch gekennzeichnet, dass** das Aktivierungslicht (3) 0,05 bis 0,5 Sekunden vor dem Entstehen der Lichtmanifestation aufleuchtet, vorzugsweise mit einer Verzögerung von bis zu 0,5 Sek. bezüglich der Anweisung zur Zündung des Lichtbogens.

15. Verfahren für die beschleunigte Verdunkelungsreaktion des optischen Elements an der persönlichen Schweißschutzvorrichtung nach einem jeden der Patentansprüche 10 bis 14, **dadurch gekennzeichnet, dass** die Steuerung des Aktivierungslichts (3) ein Zeitintervall zwischen dem Aufleuchten des Aktivierungslichts (3) und der Lichtmanifestation des Lichtbogenschweißens berechnet und die Verzögerung des Aufleuchtens des Aktivierungslichts (3) auf Basis eines rechnerischen Werts derart regelt, dass in einem folgenden Schritt des Schweißens die gewünschte Verzögerung erreicht wird, und zwar vorzugsweise eine Verzögerung von maximal 0,1 Sek.

16. Verfahren für die beschleunigte Verdunkelungsreaktion des optischen Elements an der persönlichen Schweißschutzvorrichtung nach einem jeden der Patentansprüche 10 bis 15, **dadurch gekennzeichnet, dass** das Aktivierungslicht (3) die Strahlung mit Wellenlänge zwischen 700 und 1850 nm und mit Frequenzzwischen 5 und 250 Hz abgibt.

## Revendications

1. Système pour une réaction accélérée d'obscurcissement d'un élément optique dans un équipement de protection individuellepour le soudage apte à protéger la vue, comprenant un capteur optique (1), une ouverture avec un élément optique (2) avec une perméabilité réglable pour la limitation de la diffusiond'une radiation qui dérive d'une manifestation lumineuse jusqu'à la vue, où le capteur optique (1) pour la détection de la manifestation lumineuse d'un soudage à l'arc électrique est disposé à l'intérieur de l'équipement de protection individuelle pour le soudage et le capteur optique (1) est relié à une commande de l'élément optique (2), comprenant une lumière d'activation (3) qui émet des radiations détectées par le capteur optique (1), où la lumière d'activation (3) se trouve dans le rayon d'action du capteur optique (1), **caractérisé en ce que**
ledit capteur optique (1) est le même capteur qui est utilisé à l'intérieur de l'équipement de protection individuelle pour le soudage pour détecter la manifestation lumineuse du soudage à l'arc électrique même,
où la lumière d'activation (3) est commandée par un interrupteur (4) qui est relié à l'activation du soudage à l'arc électrique de manière à ce que l'interrupteur (4) est actionné avant la transmission d'un courant électrique entre une électrode et un matériel soudé.

2. Système pour la réaction accélérée d'obscurcissement de l'élément optique de l'équipement de protection individuelle pour le soudage selon la revendication 1, **caractérisé en ce que** la lumière d'activation (3) comprend au moins une diode LED à infrarouge, préférablement comprend une pluralité de diodes LED avec des caractéristiques d'émission différentes.

3. Système pour la réaction accélérée d'obscurcissement de l'élément optique de l'équipement de protection individuelle pour le soudage selon la revendication 1 ou 2, **caractérisé en ce que** la lumière d'activation (3) appartient à un corps qui est indiqué au raccordement à un pistolet de soudage (5), où préférablement le corps est doté d'éléments d'adaptation, d'un alimentateur et d'un interrupteur (4), où le corps présente une forme annulaire et la lumière d'activation (3) est dotée d'un diffuseur optique (8).

4. Système pour la réaction accélérée d'obscurcissement de l'élément optique de l'équipement de protection personnelle pour le soudageselon la revendication 1 ou 2, **caractérisé en ce que** la lumière d'activation (3) appartient au corps du pistolet de soudage (5) et l'interrupteur (4) est un interrupteur du pistolet de soudage (5) et préférablement l'interrupteur (4) est un interrupteur à deux phases.

5. Système pour la réaction accélérée d'obscurcissement de l'élément optique de l'équipement de protection individuelle pour le soudage selon l'une quelconque des revendications de 1 à 4, **caractérisé en ce qu'**il comprend un générateur (6) de fréquence de l'alimentateur de la lumière d'activation (3), où préférablement le générateur (6) de fréquence est réglable, et **en ce qu'**il comprend en outre un élément réglable(7) du retard de la lumière d'activation (3) par rapport à l'allumage de l'interrupteur (4) et un élément pour le réglage de la période d'éclairage de la lumière d'activation (3).

6. Système pour la réaction accélérée d'obscurcissement de l'élément optique de l'équipement de protection individuelle pour le soudage selon l'une quelconque des revendications de 1 à 5, **caractérisé en ce qu'**il comprend un élément pour la détection de la manifestation lumineuse qui est relié à la commande de la lumière d'activation (3) et **en ce qu'**il comprend préférablement même un deuxième capteur optique (9), où la lumière d'activation (3) comprend un bloc pour l'extinction de la lumière d'activation (3) après la création de la manifestation lumineuse ou comprend un bloc pour l'adaptation du retard de l'accélération de la lumière d'activation de l'interrupteur (3) par rapport à l'allumage de l'interrupteur (4) ou un bloc pour omettre l'allumage de la lumière d'activation (3) tous les deux allumages de l'interrupteur (4) ou un bloc pour l'allumage de la lumière d'activation (3) en cycles calculés en fonction d'un parcours précédent d'un processus technologique.

7. Système pour la réaction accélérée d'obscurcissement de l'élément optique de l'équipement de protection individuelle pour le soudage selon l'une quelconque des revendications 1, 2, de 4 à 6, **caractérisé en ce que** l'électronique de commande (10) de la lumière d'activation (3) font partie du corps du pistolet de soudage (5) ou font partie d'une extrémité de raccordement d'un tuyau avec câbles électriques qui relient le pistolet de soudage (5) à une source de soudage, où l'électronique de commande (10) sont reliés à la lumière d'activation (3) au moyen de conducteurs électriques qui pour au moins une partie de leur longueur sont reliés à un groupe constitué par le tuyau et par les câbles électriques.

8. Système pour la réaction accélérée d'obscurcissement de l'élément optique de l'équipement de protection individuelle pour le soudage selon l'une quelconque des revendications de 1 à 7, **caractérisé en ce qu'**il comprend au moins une lumière extérieure d'activation (32) reliée à l'électronique de commande (10) au moyen d'un câble flexible, où préférablement la lumière extérieure d'activation (32) est dotée d'un élément de raccordement pour son positionnement sur un vêtement d'un opérateur.

9. Système pour la réaction accélérée d'obscurcissement de l'élément optique de l'équipement de protection individuellepour le soudage selon l'une quelconque des revendications de 1 à 8, **caractérisé en ce qu'**il comprend un élément répétiteur de signaux (11) pourvu d'un récepteur du signal optique de la lumière d'activation (3) et d'un transmetteur sous forme d'une lumière d'activation (33) pour la répétition du signal, où l'élément répétiteur di signaux (11) est adapté au positionnement indépendant à proximité du capteur optique (1).

10. Méthode pour une réaction accélérée d'obscurcissement d'un élément optique d'un équipement de protection individuelle pour le soudage apte à protéger la vue durant une opération de soudage à l'arc électrique au moyen de l'emploi du système selon l'une quelconque des revendications de 1 à 9, où une manifestation lumineuse est produite suite à la transmission d'un courant électrique entre une électrode et un matériel soudée, où ladite manifestation lumineuse est détectée par un capteur optique (1) disposé sur l'équipement de protection individuelle pour le soudage, et où sur la base d'un signal détecté par le capteur optique (1) l'obscurcissement de l'élément optique (2) est actionné, où ledit élément optique (2) avec son obscurcissement limite la pénétration d'une radiation de la manifestation lumineuse jusqu'à la vue,
où
une commande de début du soudage est détectée à l'intérieur du dispositif qui exécute le soudage à l'arc électrique avant la transmission du courant électrique entre l'électrode et le matériel soudé ;
une lumière d'activation (3) qui se répand dans un champ visuel du capteur optique (1) disposé sur l'équipement de protection individuelle pour le soudage s'allume sur la base de ladite commande, qui cause l'activation du capteur optique (1) au moyen de la lumière d'activation (3) avant que le capteur optique (1) détecte la manifestation lumineuse correspondante du soudage à l'arc électrique,
où la lumière d'activation (3) se répand au moins jusqu'au moment où le capteur optique (1) de l'équipement de protection individuelle pour le soudage même détecte la manifestation lumineuse du soudage à l'arc électrique.

11. Méthode pour la réaction accélérée d'obscurcissement de l'élément optique de l'équipement de protection individuelle pour le soudage selon la revendication 10, **caractérisée en ce qu'**après la détection de la manifestation lumineuse par le capteur optique correspondant (1) disposé sur l'équipement de protection individuelle pour le soudage la lumière d'activation (3) est éteinte par le système.

12. Méthode pour la réaction accélérée d'obscurcissement de l'élément optique de l'équipement de protection individuelle pour le soudage selon la revendication 10 ou 11, **caractérisée en ce que** la commande d'allumage de la lumière d'activation (3) durant le soudage à l'arc électrique en atmosphère protectrice est donnée durant un temps de pré-gaz, où un gaz protecteur est transféré à un point de soudage avant l'allumage de l'arc électrique.

13. Méthode pour la réaction accélérée d'obscurcissement de l'élément optique de l'équipement de protection individuelle pour le soudage selon la revendication 10 ou 12, **caractérisé en ce que** la lumière d'activation (3) se répand pour un temps préétabli à partir du moment de son allumage, préférablement pendant au moins 1 seconde, ou la lumière d'activation (3) s'éteint après que sa commande a reçu l'information sur la détection de la manifestation lumineuse, où préférablement elle reçoit ladite information par le deuxième capteur optique (9).

14. Méthode pour la réaction accélérée d'obscurcissement de l'élément optique de l'équipement de protection individuelle pour le soudage selon l'une quelconque des revendications de 10 à 13, **caractérisée en ce que** la lumière d'activation (3) s'allume de 0,05 à 0,5 secondesavant la création de la manifestation lumineuse, préférablement elle s'allume avec un retard de 0,5 secondes au maximum par rapport à la commande d'allumage de l'arc électrique.

15. Méthode pour la réaction accélérée d'obscurcissement de l'élément optique de l'équipement de protection individuelle pour le soudage selon l'une quelconque des revendications de 10 à 14, **caractérisée en ce que** la commande de la lumière d'activation (3) calcule un intervalle de temps entre l'allumage de la lumière d'activation (3) et la manifestation lumineuse du soudage à l'arc électrique et règle le retard de l'allumage de la lumière d'activation (3) sur la base d'une valeur calculée, de manière à ce que dans une phase de soudage successive l'on obtient le retard souhaité, où préférablement le retard sera de 0,1 seconde au maximum.

16. Méthode pour la réaction accélérée d'obscurcissement de l'élément optique de l'équipement de protection individuelle pour le soudage selon l'une quelconque des revendications de 10 à 15, **caractérisée en ce que** la lumière d'activation (3) émet la radiation avec une longueur d'onde comprise entre 700 et 1850 nm etavec une fréquence comprise entre 5 et 250 Hz.
